(19) Europäisches Patentamt
European Patent Office
Office européen des brevets

(11) EP 1 295 621 B1

(12) **EUROPEAN PATENT SPECIFICATION**

(45) Date of publication and mention
of the grant of the patent:
**07.11.2018 Bulletin 2018/45**

(51) Int Cl.:
*A61M 16/10* (2006.01)     *A61M 16/08* (2006.01)
*A61M 16/16* (2006.01)

(21) Application number: **02020964.9**

(22) Date of filing: **19.09.2002**

(54) **Humidified gases delivery apparatus**

Vorrichtung zur Abgabe befeuchteter Gase

Dispositif d'administration de gaz humidifié

(84) Designated Contracting States:
**AT BE BG CH CY CZ DE DK EE ES FI FR GB GR
IE IT LI LU MC NL PT SE SK TR**

(30) Priority: **19.09.2001 NZ 51431401**

(43) Date of publication of application:
**26.03.2003 Bulletin 2003/13**

(73) Proprietor: **Fisher & Paykel Healthcare Limited
East Tamaki,
Auckland (NZ)**

(72) Inventors:
• **Smith, Daniel John
Mt. Albert,
Auckland (NZ)**

• **Kadhum, Hussein
Hillsborough,
Auckland (NZ)**

(74) Representative: **Hoarton, Lloyd Douglas Charles
et al
Forresters IP LLP
Skygarden
Erika-Mann-Strasse 11
80636 München (DE)**

(56) References cited:
**EP-A- 0 556 561      EP-A- 1 138 341
WO-A-02/32486      DE-A- 3 311 811
DE-C- 3 629 353      GB-A- 2 277 689
US-A- 4 780 247      US-A1- 2002 124 847**

## Description

## BACKGROUND TO THE INVENTION

### Field of the Invention

The present invention relates to conduits for humidified breathing circuits.

### Summary of the Prior Art

A number of methods are known in the art for supplying humidified gases to a patient requiring breathing assistance. Such prior art humidifiers generally comprise a source of pressurised air (or other mixture of gases), a humidification chamber including a source of water and a heating means to vaporise the water, and a conduit to convey the humidified gases to the patient or user.

For example US patent 4,038,980 describes a "flash vaporisation" humidifier where water drips onto a low thermal mass heater to create respiratory humidity. It mentions "control means may be provided automatically to regulate the water supply rate in response to means sensing the relative humidity", however they prefer a manual control of water flow rate. Thus it incorporates a humidity sensor and controls the water rate, as opposed to controlling the amount of electrical heating.

US patent 5,092,326 also describes the use of a humidity sensor in a humidifier. It describes a high frequency ventilation system that incorporates a heated humidifier and a humidity sensor, where these are linked to a central microprocessor. Apparatus is disclosed to moisten a gas mixture supplied to the airway, and a microprocessor controls the amount of moisture supplied to the gas mixture.

US patent 5,769,071 describes a humidifier incorporating a heat and moisture exchanger (HME), supply of water to the HME, heater element and humidity sensor. The humidity sensor can control humidity via water supply rate or temperature (via the heater element). The humidity sensor is described as being at the patient airway.

US patent 5,988,164 describes a heated breathing tube system for use with a humidifier. This uses a relative humidity sensor (located near the patient) to control the amount of heating provided by the heated breathing circuit so that the gas is at a constant level of relative humidity. The heated breathing circuit may use either electrical heating, or heating via warm recirculating water in a tube. Also described is a method of control of the electric heater wire or heated water tube based on the output of relative humidity sensor.

The previously mentioned US patents 4,038,980 and 5,769,071 both describe humidifiers where the humidification chamber is located close (proximal) to the patient. These have the disadvantage of introducing weight, heat and complexity near the patient which is inconvenient and could be painful to the patient.

Of the cited prior art only US patent 5,988,164 specifically describes the humidification chamber as being located remotely from the patient.

European patent application EP-A-1138341, which is state of the art under Article 54(3) EPC, discloses a heated gases delivery tube that self-regulates the temperature along its length. The heater is made from separate sections of PTC material, arranged in series along the length of the delivery tube, and linked by two heater wires that run longitudinally along the length of the heater.

EP 0 556 561 A1 describes a heated breathing gas tube for a medical ventilator. The tube is made of flexible material and is reinforced by a plastic spiral around the outside. This prevents the tube from kinking and stopping the airflow. The heater wires are positioned on the outside of the tube, each side of the spiral. The inside of the spiral carries a return lead for the heaters. The heaters are connected in parallel, with the return connection attached at the free end of the tube.

GB 2 277 689 A describes a heater for a device (such as a filter or HME device) through which gas flows in a breathing gas circuit. The heater comprises an electrical resistance heating element having a surface which is in thermal contact with a wall of the device such that its internal surface can be heated to reduce condensation within the device. The heater is separable from the device and contacts the external surface of the wall of the device. In one example, the heater may include a positive temperature coefficient resistance so that the temperature of the heater is self regulating.

There are several disadvantages of the prior art systems using a humidification chamber located remotely from the patient. It is normally assumed that gases leaving such prior art humidifiers are saturated with water vapour (100% relative humidity). However there is no guarantee that the gases leaving such humidifiers are in fact saturated with water vapour. In certain circumstances (e.g. with the incoming air already warm), the gases leaving such humidifiers can be significantly less than 100% relative humidity. This is because the humidifiers are typically controlled to achieve a desired outlet gas temperature, which in some cases may not be much more than the incoming air.

Another drawback of the prior art systems is that condensation can occur in the (sometimes heated) conduits connecting the patient to the respiratory assistance equipment. This may occur if the temperature profile along such conduits is not even and allows some parts of the conduit to be colder than the gas at these points.

A third disadvantage of such prior art systems is that where the gas leaving the humidifier is at 100% relative humidity it must be heated immediately by some form of conduit heater or it may lose heat through the walls of the conduit otherwise condensation and therefore a drop in the amount of absolute humidity contained in the gas will result.

A fourth disadvantage of the prior art systems is the need for a sensor very near to the patient, which adds to the weight and bulk of equipment at the patient's airway.

## SUMMARY OF THE INVENTION

It is therefore an object of the present invention to provide an inspiratory conduit which goes some way to overcoming the above-mentioned disadvantages.

In a first aspect the invention may broadly be said to consist in a gases transportation pathway for transporting humidified gases to a patient, said pathway comprising an enclosing wall that includes at least a layer of positive temperature coefficient material,

wherein the localised electrical resistance of said material is positively related to the localised temperature, at least two electrical conductors running the length of said pathway, disposed in electrical contact with said positive temperature coefficient material,

wherein

said conductors are arranged helically along said gases transportation pathway

and are at least substantially constantly spaced from one another along the length of said gases transportation pathway.

In a further aspect the invention may broadly be said to consist in a humidification apparatus for providing a humidified gases flow to a patient or other person in need of such gases comprising:

humidification chamber means having an inlet and an outlet to allow said gases flow to pass through said humidification chamber means,

chamber heating means provided adjacent said humidification chamber means adapted to vaporise liquid water in said humidification chamber means in order to provide water vapour to said gases flow passing through said humidification chamber means,

and a gases transportation pathway as described above connected to said outlet of said humidification chamber means to convey said gases flow to said patient or other person in need of such gases.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

The invention consists in the foregoing and also envisages constructions of which the following gives examples.

## BRIEF DESCRIPTION OF THE DRAWINGS

One preferred form of the present invention will now be described with reference to the accompanying drawings.

Figure 1 shows an example of an humidification system, with three basic parts

Figure 2 is a plan view of a section of a ribbon of PTC material with an electrode embedded along each edge.

Figure 3 is a plan view of a spirally configured heater element using the PTC ribbon of Figure 2.

Figure 4 is a plan view of a second form of spirally configured PTC ribbon heater element.

Figure 5 is perspective view of a tube not embodying the present invention formed with a spirally wound PTC ribbon (without pre-embedded conductors) with longitudinally oriented conductors in the tube.

Figure 6 is a plan view of a section of a ribbon of PTC material with a conductor embedded along one edge and second conductor embedded near the centre.

Figure 7 is a plan view of a spiral forming arrangement performing a conduit using the ribbon of Figure 6 (with the forming mandrel not shown).

Figure 8 shows construction of a tube not embodying the present invention incorporating flexible PTC elements in a parallel wire configuration.

Figure 9 shows a humidifier configuration using the tube in Figure 8.

## DETAILED DESCRIPTION OF THE PREFERRED EMBODIMENTS

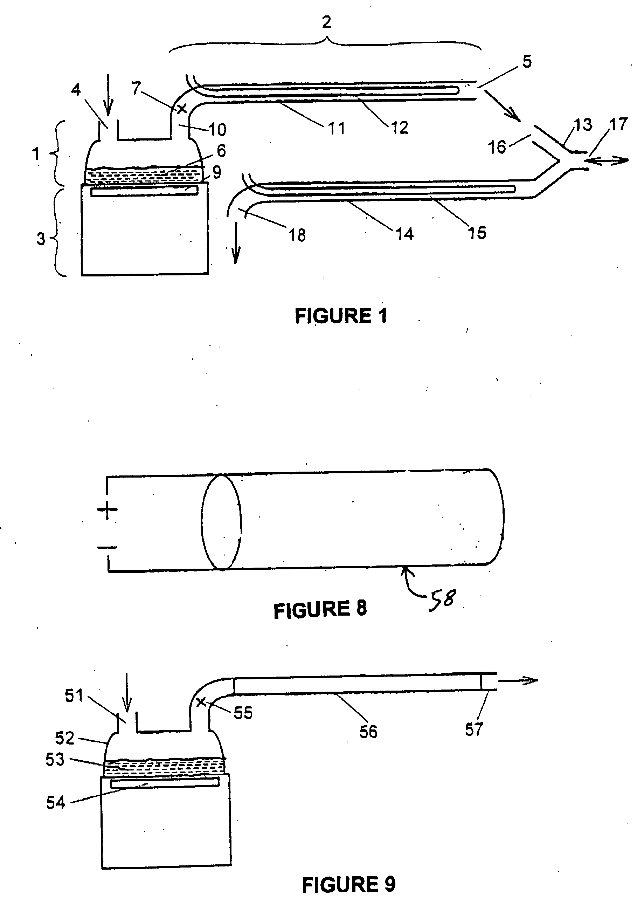

Figure 1 illustrates a typical respiratory humidification system, comprised of three parts:

1) a humidification chamber located at a distance from the patient, which heats and substantially saturates gases flowing through it;

2) a delivery system consisting of a flexible tube which carries humidified gases from the humidification chamber 1 to the gas outlet 5 ; and

3) a heater base which heats the humidification chamber 1 and provides measurement and control functions.

The gas to be humidified flows into the chamber 1 from port 4 and leaves the delivery system 2 at gas exit port 5. Gas from exit port 5 flows to a patient via a face mask or similar (not shown). Dry gases at the gas input 4 are heated and humidified by passing over the surface of hot water 6 in the chamber 1 so that they are substantially saturated with water vapour when they leave chamber 1 at exit port 10. Hot water 6 is heated by heater plate 9 and the amount of heating is controlled so that the gas reaches a predetermined temperature at exit port 10. Therefore the humidification chamber 1 acts to heat and humidify the medical gases so that they are substantially saturated at the output of chamber 1, and are at a predetermined temperature.

The gas delivery system 2 (also known as a delivery tube or breathing circuit) consists of a flexible tube 11 containing a heater 12. The gas from the humidification chamber 1 passes through the tube 11 and is heated by heater 12 to offset heat losses through the walls of tube 11.

The system as described has gas entering gas inlet 4 from a continuous flow gas source (not shown) and exiting the system through gas outlet 5. However the system is equally applicable where the gas source is a ventilator, which creates intermittent flow patterns to provide breaths to a patient. In this case gas outlet port 5 is connected directly to gas inlet port 16. The patient is connected to port 17 via an endotracheal tube, mask, mouthpiece or other patient interfaces (not shown). During patient inspiration dry gases from the ventilator enter the system at inlet port 4, pass through chamber 1, delivery system 2, pass through wye-piece 13 and reach the patient through port 17. During patient exhalation gases pass back through port 17, through wye-piece 13, tube 14 and leave through gas outlet port 18. Tube 14 may also be heated by heater 15 to prevent condensation.

The present invention provides a heated delivery tube which self-regulates its temperature at a desired level. The heater may be embedded in the wall of the delivery tube itself, form the fabric of the tube or lie inside the lumen of the delivery tube. The heater of the present invention is formed from a positive temperature coefficient (PTC) material.

The resistance of a PTC material increases markedly once it reaches a threshold temperature, resulting in reduced power consumption and subsequent cooling. The delivery tube may pass through more than one environment, or may have localised drafts present on certain parts of the tube.

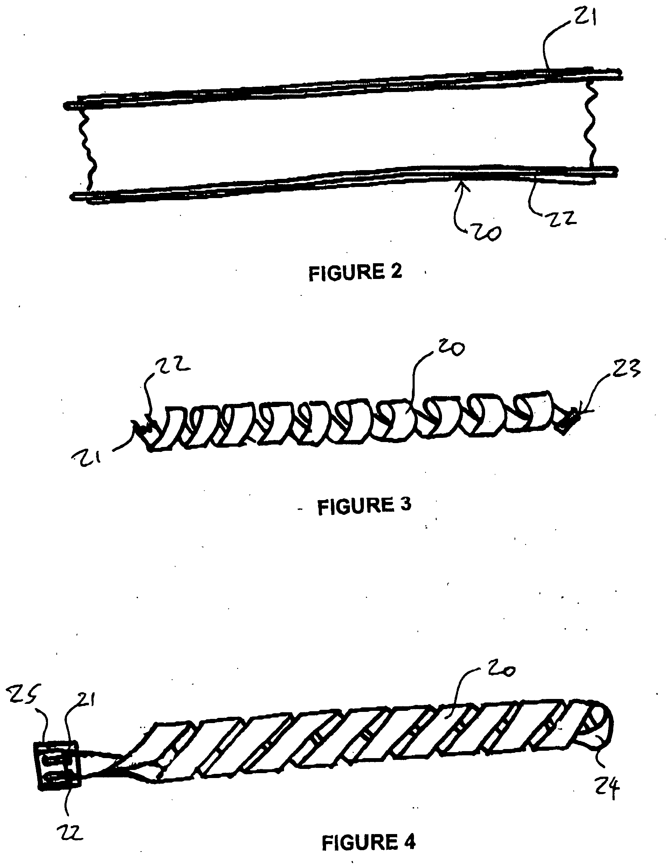

In one embodiment of the present invention the PTC heater is provided as an elongate structure laying within the lumen of the delivery tube. The construction according to a preferred embodiment is illustrated with respect to Figures 2 to 4. In particular the heater structure is formed from a ribbon 20 of PTC plastic material with conductors 21, 22 embedded in the plastic material adjacent the opposite edges thereof. In use the conductors are attached to a power supply to provide a voltage difference between the conductors and cause a current to flow between them depending on the resistance of the PTC material.

The ribbon may be provided in the tube as a single length of ribbon blindly terminated at one end and terminated with a power connector at the other end. This configuration is illustrated in Figure 3 where the ribbon 20 is wound into a generally helical configuration and is terminated at one end with a blind connector 23. Termination of the other end at a power connector is not shown. In an alternative configuration the ribbon may be provided as a loop so that both ends terminate at the power connector with both ends of the positive electrode terminating at the positive pin and both ends of the negative or ground electrode terminating at the ground and negative pin. This configuration is depicted in Figure 4, in which the ribbon 20 is provided in a generally double helical configuration. The conductors 21 and 22 have both ends terminating in the power connector 25 at one end of the heater structure. The ribbon 20 loops back upon itself at the other end 24 of the heater structure.

With the pair of conductors provided along opposite edges of the ribbon the PTC material offers an amorphous array of parallel current paths along the entire length of the ribbon. Where the internal conduit temperature is lower the heater structure will have a lower resistance and more current will flow producing a greater heater effect. Where the internal temperature in the conduit is higher the PTC material will have a higher resistance, choking off current flow and reducing heating in that region of the conduit.

In a further aspect of the invention the PTC material is arranged in a parallel circuit over the length of the tube and forming part of the wall itself the full benefit of using PTC heater can be obtained. At the cold portions of the tube the material will have a lower resistance, which will result in more heat being dissipated in that area. Thus the tube will tend to regulate its own temperature.

In particular if the PTC material is composed to provide a threshold temperature at or just above the preferred gases temperature (eg above the dew-point of the humidified gases) the PTC material will maintain itself at that threshold temperature (with some hysteresis fluctuation) and condensation on the conduit surface will be at least substantially eliminated. This provides effective condensation control then maintaining an elevated temperature for the humidified gases where condensation may still form on the cold wall surfaces.

PTC material behaviour is exhibited in a range of polymer compositions with electrically conductive fillers. The behaviour can be characterised by a general statement that "providing certain other conditions are fulfilled, the composition becomes electrically conductive when particles of electrically conductive filler form a continuous chain, penetrating the material from the point of entry of electric current to the place where it leaves the polymer material". Polymer compositions containing electrically conductive filler can exhibit PTC properties due to the formation of a chain of filler particles that are close enough for current to flow at a certain temperature, generating heat which increases the temperature of the material until it reaches a phase transformation temperature. At the phase transformation temperature the crystalline polymer matrix changes to an amorphous structure. This change is accompanied by a small thermal expansion, forcing filler particles to move apart, breaking the conductive paths. Accordingly resistance rises sharply at this phase transformation temperature. As the material cools the small thermal conduction allows new conductive paths to form and current flow to resume. The rise and fall in temperature and the thermal contraction and expansion provides an inherent hysteresis in the cycle.

In producing a PTC material a number of factors have a bearing on the performance of the material. Particular factors include the quantity, type and particle size of the carbon black (or other conductive filler) used in the composite, the polymer that the carbon black binds with during mixing of the base materials and the process conditions such as temperature, pressure and time of mixing. It is important that the conductive filler particles are distributed evenly through the composite so that the composite exhibits uniform PTC behaviour.

For the present invention a PTC material having a phase transformation temperature not exceeding 40°C is desired. One composition meeting these criteria has been developed and has the following composition:

- 20% by weight carbon black powder having a surface area of $254m^2/g$ and oil Di-Butyl- Phthalate absorption of $188cm^3/100g$. This powder is available as VULCAN XC-72 (powder) from Cabot Corporation.
- 64% Ethylene-Vinyl-Acetate. This material is available as ELVAX (grade 40w) from Dupont (E.I. du Pont de Nemours and Company), with a density of 965kg per $m^3$, a melting point of 46°C and melting index of 52.
- 13.5% Plastomer. An example plastomer is available as EXACT 2M055 from ExxonMobil Corp, having a density of $882kg/m^3$, a melting point of 70°C and a melting index of 3.
- 2.5% Wax.

This material was uniformly mixed and extruded to form a PTC ribbon with embedded conductors using a segmented screw extruder. The composite performance showed an acceptable level of self regulation without the temperature exceeding 40°C.

Varying the amount of carbon black up or down within this composition has the effect of varying the phase transition temperature. Where delivery of humidified gases to a patient a phase transition temperature in the range 30°C to 45°C may be appropriate, with the particular transition temperature required depending on the particular humidified gases treatment to be delivered. For example humidified gases delivery is being promoted for the treatment of chronic obstructive pulmonary disease (COPD). Whereas treatment humidified gases are delivered to the patient, usually via a nasal cannular, at between 35 and 45°C. Therefore for treatment of COPD a PTC material having a phase transition temperature in the range 35°C to 45°C is preferred. Similarly humidified gases are being promoted for use in patient insufflation for surgical procedures. In this application humidified gases are delivered at a temperature between 35°C and 40°C and accordingly a PTC material having a phase transition temperature in this range is preferred. This is also the preferred range for the temperature of humidified gases for respiration of an intubated patient, and therefore the preferred phase transition temperature for the PTC material used in manufacturing a conduit for that purpose. However where a patient is receiving humidified respiratory gases via a face mask it has been found that a somewhat lower delivery temperature is preferable, in the range 35°C to 35°C. Accordingly for the manufacture of conduits for delivery of respiratory gases to a patient via a face mask a PTC material phase transition temperature of 30°C to 35°C is preferred.

The applicant has found that there are many ways of producing a tube having a PTC wall material with a pair of conductors running the length of the tube to have all of the potential pathways through the PTC material operating in parallel. A number of preferred embodiments are now described which are particularly advantageous.

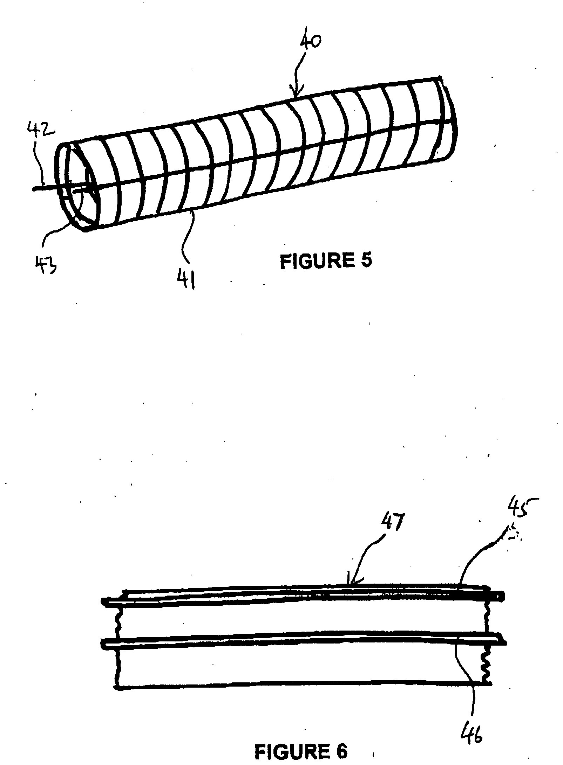

With reference to Figure 5 a smooth walled tube 40 not embodying the present invention is shown by way of a first example. The smooth walled tube 40 has a PTC plastic material extruded as a narrow and thin ribbon 41 and wound helically with overlapping edges of adjacent turns. The edges of adjacent turns bound firmly to one another, fusing together in their moulten state. A pair of conductors 42, 43 run longitudinally in the tube wall. The conductors are diametrically opposed. The conductors may be applied to either the internal or external surfaces of the molten PTC material during forming of the tube. To apply the conductors to the internal surface the conductors are applied longitudinally to the forming mandrel prior to laying the extruded PTC ribbon in place. Alternatively they may be applied directly to the outside of the PTC material while the material is still in a molten state. With the claimed invention, these conductors are applied helically rather than in a straight longitudinal direction, and multiple conductors may be used.

Design of a PTC tube of this type involves selection of a wall thickness, a conductor gauge and a density of conductors in the PTC tube wall. The total resistance R (OMEGA) of the tube wall in its pre-threshold state will be a measure of the available power output for a given voltage. The available power output must be sufficient to offset the heat lose from the tube to its surrounding environment and (if the gases are entering the tube in a cooler state) to the humidified gases. The total resistance is proportional to the pre-threshold volume resistivity X ($\Omega$ m) of the material and to the average shortest path distance between the conductors of opposite plurality. The total resistance is also proportional to the inverse of the length $L_C$(m) of the conductors and to the inverse of the wall thickness t (m) of the PTC material. Furthermore, typically there will be a pair of opposite and alternate paths for current to flow from a conductor of one polarity to the conductor of the other polarity, halving the total resistance. Thus the total resistance can be found from the formula:

$$R = \frac{X\overline{w}}{2 L_c t}$$

where $\overline{w}(m)$ is the average shortest length path between conductors.

Therefore for a given tube length and diameter the total cold resistance may be varied by varying the density of conductors (varying the average shortest path distance between conductors) or by varying the wall thickness. The density of conductors may be varied by adding additional conductors in parallel (eg: a second or more pair of conductors) or by disposing the conductors in a helical arrangement with decreasing pitch corresponding to an increased density. For a given tube diameter D (m) and tube length $L_t$ (m) then the average shortest path length can be found using the total conductor path length for a single polarity (half the total conductor length) by:

$$\overline{w} = \frac{\pi D L_T}{2 L_c}$$

The tube of Figure 5 may be reinforced by applying a spiral bead, or by applying circumferential ribs to the outside of the tube, or by corrugating the tube, or by adding additional layers to the tube, particularly of a spiral ribbed or corrugated configuration, which would also provide additional external insulation from the ambient conditions.

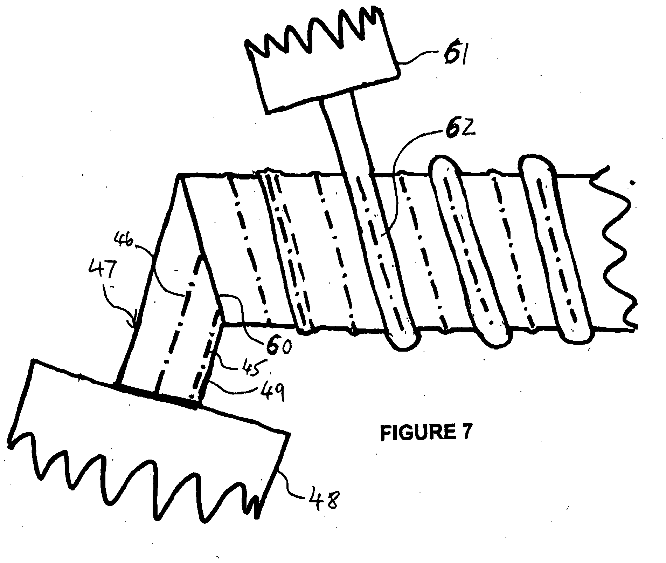

A further construction is illustrated in Figures 6 and 7. Figure 6 shows a pair of conductors 45, 46 extruded into a ribbon of PTC material. The first conductor 45 is disposed adjacent one edge of the PTC ribbon 47. The second conductor 46 is disposed adjacent the centre of the PTC ribbon 47. The exact location of the conductors within the PTC material is not critical, however the spacing between the conductors should be half of the pitch of winding the ribbon on to the former (eg; (width of ribbon - width of overlap between turns) DIVIDED 2). For additional conductor density, additional pairs of conductors may be used. For lower conductor density the width of ribbon may be increased or alternatively a single conductor may be provided in the ribbon but two ribbons may be extruded and wound on to the former as a double helix.

Referring to Figure 7 a manufacturing configuration is shown (without the rotating former, which may for example be a spiral pipeline mandrel available from OLMAS SRL of Italy). In this manufacturing configuration the PTC ribbon 47 is co-extruded with the embedded pair of conductors 45, 46 by a first extruder head 48. It is extruded directly on the former at an angle corresponding to the pitch of the former (the relationship between the advance and rotation speeds of tubes formed on it). The ribbon 47 is laid on the former so that the leading edge 49 of each new lap overlaps the trailing edge 60 of the immediately preceding turn. A reinforcing bead 62 is preferably extruded on to this overlap by an additional extruder head 61. The reinforcing bead 62 assists the bonding between overlapping turns of the ribbon as well as providing reinforcing against crushing of the formed tube.

Alternatively a conduit may be formed on a spiral pipeline mandrel with the reinforcing bead extruded to lie between the overlap of turns of the ribbon. This is particularly suited to where the ribbon is preformed and will not bond to itself without assistance. In this case contact may be provided between adjacent turns of the PTC ribbon along either side of the bead (for example by extended overlap) or the ribbon used may be have a conductor along each edge (as in Figure 2).

Figure 8 shows a further construction of a tube not embodying the present invention incorporating flexible PTC elements in a parallel wire configuration. The tube 58 is a flexible PTC material, which has two conductors built into it.

The tube 58 according to this construction may be a directly extruded tube with the conductors co-extruded into the tube wall, or alternatively the conductors may be added subsequent to forming the tube by direct application to the exterior of the tube as wires or as conductive ink.

The tube may have an outer layer (not shown) which provides electrical insulation and thermal insulation to the tube.

The tube may be corrugated by passing through a set of corrugating rollers, to provide flexibility and lateral reinforcing against crushing.

The PTC design could also be extended to incorporate PTC heaters in other parts of the patient breathing circuit, such as the flexible extension tube which is usually connected between the Y-piece (port 17 of Figure 1) and the patient's endotracheal tube. A further extension of the PTC tube concept would be into a self-heated and temperature controlled endotracheal tube.

The tube with PTC wall material allows a humidifier to be used without any sensor at the patient airway. Figure 9 shows a humidifier configuration using a PTC tube. Gas enters humidification chamber 52 via inlet port 51 and is humidified by water 53, heated by heater plate 54. An absolute humidity sensor 55 controls the heater plate so that the gas passing sensor 55 is at a desired level of absolute humidity. PTC tube 56 is heated by an external voltage (not shown) so that the internal surface temperature is at a constant desired temperature, which is selected to be above the dewpoint of the gas. The gas which leaves tube 56 at outlet 57 will therefore be near the temperature of the tube, and containing the desired level of absolute humidity which was controlled by absolute humidity sensor 55.

**Claims**

1. A gases transportation pathway (2) for transporting humidified gases to a patient, said pathway (2) comprising an enclosing wall (11) that includes at least a layer of positive temperature coefficient material (12), wherein the localised electrical resistance of said material is positively related to the localised temperature;
at least two electrical conductors (21, 22, 45, 46) running the length of said pathway, disposed in electrical contact with said positive temperature coefficient material,
wherein
said conductors (21, 22, 45, 46) are arranged helically along said gases transportation pathway (2) and are at least substantially constantly spaced from one another along the length of said gases transportation pathway (2).

2. A gases transportation pathway as claimed in claim 1 wherein said enclosing wall (11) includes at least one helically arranged ribbon (47) of positive temperature coefficient material with adjacent turns of said ribbon (47) overlapping and fused to one another.

3. A gases transportation pathway as claimed in claim 2 wherein said conductors (45, 46) are disposed within said ribbon (47) of positive temperature coefficient material, in constant position in relation to said ribbon (47).

4. A gases transportation pathway (2) as claimed in any one of claims 1 to 3 wherein said positive temperature coefficient (12) material has a phase transformation temperature between 35°C and 40°C.

5. A gases transportation pathway (2) as claimed in claim 4 wherein said PTC material comprises a conductive filler uniformly distributed within a polymer composition, said conductive filler comprising approximately 20% by weight carbon black powder.

6. A humidifier apparatus (1, 2, 3) for providing a humidified gases flow to a patient or other person in need of such gases comprising:

a humidification chamber (1) having an inlet (4) and an outlet (10) to allow said gases flow to pass through said humidification chamber (1),
a chamber heating means (3) provided adjacent said humidification chamber (1) adapted to vaporise liquid water in said humidification chamber (1) in order to provide water vapour to said gases flow passing through said humidification chamber (1),
and

a gases transportation pathway (2) as claimed in any one of claims 1 to 5 connected to said outlet of said humidification chamber (1) to convey said gases flow to said patient or other person in need of such gases.

7. A humidification apparatus as claimed in claim 6 wherein said positive temperature coefficient material has a phase transformation temperature within 5°C of the temperature of said gases flow leaving said humidification chamber (1).

8. A humidification apparatus as claimed in either claim 6 or claim 7 wherein the phase transformation temperature of said positive temperature coefficient material is above the temperature of said gases flow exiting said humidification chamber (1) and below 40°C.

**Patentansprüche**

1. Gastransportweg (2) zum Transportieren befeuchteter Gase zu einem Patienten, wobei der Weg (2) eine umschließende Wand (11) umfasst, die mindestens eine Schicht aus einen positiven Temperaturkoeffizienten aufweisendem Material (12) beinhaltet, worin der lokalisierte elektrische Widerstand des Materials positiv mit der lokalisierten Temperatur zusammenhängt;
mindestens zwei entlang des Wegs verlaufende elektrische Leiter (21, 22, 45, 46), die in elektrischem Kontakt mit dem einen positiven Temperaturkoeffizienten aufweisenden Material angeordnet sind,
worin die Leiter (21, 22, 45, 46) spiralförmig entlang des Gastransportwegs (2) angeordnet sind und mindestens im Wesentlichen konstant voneinander entlang der Länge des Gastransportwegs (2) beabstandet sind.

2. Gastransportweg nach Anspruch 1, worin die umschließende Wand (11) mindestens ein spiralförmig angeordnetes Band (47) aus einen positiven Temperaturkoeffizienten aufweisendem Material beinhaltet, wobei nebeneinanderliegende Windungen des Bandes (47) überlappen und miteinander fusioniert sind.

3. Gastransportweg nach Anspruch 2, worin die Leiter (45, 46) innerhalb des Bandes (47) aus einen positiven Temperaturkoeffizienten aufweisendem Material, in konstanter Position in Bezug auf das Band (47), angeordnet sind.

4. Gastransportweg (2) nach einem der Ansprüche 1 bis 3, worin das einen positiven Temperaturkoeffizienten aufweisende Material (12) eine Phasentransformationstemperatur zwischen 35 °C und 40 °C aufweist.

**5.** Gastransportweg (2) nach Anspruch 4, worin das PTC-Material einen leitfähigen Füllstoff umfasst, der gleichmäßig verteilt innerhalb einer Polymerzusammensetzung ist, wobei der leitfähige Füllstoff ungefähr 20 Gew.-% Rußpulver umfasst.

**6.** Befeuchtungsvorrichtung (1, 2, 3) zum Bereitstellen eines befeuchteten Gasflusses zu einem Patienten oder einer anderen Person mit Bedarf an derartigen Gasen, umfassend:

eine Befeuchtungskammer (1) mit einem Einlass (4) und einem Auslass (10), damit der Gasfluss durch die Befeuchtungskammer (1) hindurch verlaufen kann,
ein Kammerheizmittel (3), das neben der Befeuchtungskammer (1) bereitgestellt und dafür ausgelegt ist, flüssiges Wasser in der Befeuchtungskammer (1) zu verdampfen, um Wasserdampf für den durch die Befeuchtungskammer (1) hindurch verlaufenden Gasfluss bereitzustellen, und
einen Gastransportweg (2) nach einem der Ansprüche 1 bis 5, der mit dem Auslass der Befeuchtungskammer (1) verbunden ist, um den Gasfluss zu dem Patienten oder einer anderen Person mit Bedarf an derartigen Gasen zu fördern.

**7.** Befeuchtungsvorrichtung nach Anspruch 6, worin das einen positiven Temperaturkoeffizienten aufweisende Material eine Phasentransformationstemperatur innerhalb von 5 °C der Temperatur des die Befeuchtungskammer (1) verlassenden Gasflusses aufweist.

**8.** Befeuchtungsvorrichtung entweder nach Anspruch 6 oder Anspruch 7, worin die Phasentransformationstemperatur des einen positiven Temperaturkoeffizienten aufweisenden Materials oberhalb der Temperatur des die Befeuchtungskammer (1) verlassenden Gasflusses und unterhalb von 40 °C ist.

**Revendications**

**1.** Un conduit d'acheminement de gaz (2) destiné à acheminer des gaz humidifiés à un patient, ledit conduit (2) comprenant une paroi d'enceinte (11) qui inclut au moins une couche de matériau à coefficient de température positif (12), dans lequel la résistance électrique locale dudit matériau est reliée positivement à la température locale ; au moins deux conducteurs électriques (21, 22, 45, 46) situés sur la longueur dudit conduit, disposés en contact électrique avec un matériau à coefficient de température positif,
dans lequel lesdits conducteurs (21, 22, 45, 46) sont agencés de manière hélicoïdale le long dudit conduit d'acheminement de gaz (2) et sont au moins sensiblement constamment espacés l'un de l'autre le long de la longueur dudit conduit d'acheminement de gaz (2).

**2.** Un conduit d'acheminement de gaz selon la revendication 1 dans lequel ladite paroi d'enceinte (11) inclut au moins un ruban agencé de manière hélicoïdale (47) de matériau à coefficient de température positif avec des spires adjacentes dudit ruban (47) se chevauchant et fusionnées les unes avec les autres.

**3.** Un conduit d'acheminement de gaz selon la revendication 2 dans lequel lesdits conducteurs (45, 46) sont disposés dans ledit ruban (47) de matériau à coefficient de température positif, à une position constante par rapport audit ruban (47).

**4.** Un conduit d'acheminement de gaz (2) selon l'une quelconque des revendications 1 à 3 dans lequel ledit matériau à coefficient de température positif (12) a une température de transformation de phase comprise entre 35°C et 40°C.

**5.** Un conduit d'acheminement de gaz (2) selon la revendication 4 dans lequel ledit matériau PTC comprend une charge conductrice répartie uniformément dans une composition polymère, ladite charge conductrice comprenant environ 20% en poids de poudre de noir de carbone.

**6.** Un appareil humidificateur (1, 2, 3) destiné à fournir un flux de gaz humidifiés à un patient ou une autre personne ayant besoin de tels gaz, comprenant :

une chambre d'humidification (1) ayant un orifice d'entrée (4) et un orifice de sortie (10) pour permettre audit flux de gaz de passer à travers ladite chambre d'humidification (1),
un moyen de chauffage de chambre (3) fourni adjacent à ladite chambre d'humidification (1) adapté pour vaporiser de l'eau liquide dans ladite chambre d'humidification (1) afin de fournir de l'eau audit flux de gaz passant à travers ladite chambre d'humidification (1), et
un conduit d'acheminement de gaz (2) selon l'une quelconque des revendications 1 à 5, raccordé audit orifice de sortie de ladite chambre d'humidification (1) pour amener ledit flux de gaz audit patient ou autre personne ayant besoin de tels gaz.

**7.** Un appareil d'humidification selon la revendication 6 dans lequel un matériau à coefficient de température positif a une température de transformation de phase dans une fourchette de 5°C de la température dudit flux de gaz quittant ladite chambre d'humidification (1).

8. Un appareil d'humidification selon la revendication 6 ou la revendication 7 dans lequel la température de transformation de phase dudit matériau à coefficient de température positif est supérieure à la température dudit flux de gaz sortant de ladite chambre d'humidification (1) et inférieure à 40°C.

**FIGURE 1**

**FIGURE 8**

**FIGURE 9**

**FIGURE 2**

**FIGURE 3**

**FIGURE 4**

**FIGURE 5**

**FIGURE 6**

FIGURE 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4038980 A
- US 5092326 A
- US 5769071 A
- US 5988164 A
- EP 1138341 A
- EP 0556561 A1
- GB 2277689 A